# EUROPEAN PATENT APPLICATION

(11) **EP 4 537 767 A1**
(43) Date of publication of application: **16.04.2025**
(21) Application number: 24200687.2
(22) Date of filing: 17.09.2024
(51) Int. Cl.: A61B 17/00, A61B 17/04

(54) **SINGLE TUNNEL TISSUE REPAIRS**

(30) Priority: 19.09.2023 US 202363583594 P
(71) Applicant: Arthrex, Inc., Naples, FL 34108-1945 (US)
(72) Inventor: HEGG, David J., Naples, FL 34109 (US); FEENEY, Mark D., Naples, FL 34119 (US)
(74) Representative: Lohr, Jöstingmeier & Partner

(57) **Abstract**

Knotless constructs and methods of tissue repairs are disclosed herein.

## Description

### Description of the related art

Current techniques for attaching soft tissue to bone require two tunnels in a bone for creating a boney bridge to tie knots over for final fixation of soft tissue to bone.

### Summary

The disclosure herein relates to the field of surgery and, more specifically, to knotless suture constructs and associated methods of tissue repairs. The problem to be solved is to provide surgical construct for tissue repair, which is bone-preserving and requires one bone tunnel, in that it eliminates the need for two or more bone tunnels to create a bone bridge for tying knots for final fixation of soft tissue to bone.

Solutions of the problem are described in the independent claims. The dependent claims relate to further improvements.

Knotless constructs, surgical systems, assemblies, and methods of tissue repairs are disclosed. A construct can create a knotless repair. In an embodiment, a construct as described herein can be self-locking, tensionable, and bone-preserving.

A construct can include fixation devices in the form of an all-suture, knotless, and tensionable implant through a single stepped bone tunnel for tissue fixation. A securing material can be attached to the all-suture implant to be employed as a bridge for tensioning and final fixation of the repair. A securing material can be a length of material, for example, a fixed length of suture such as a suture sheath. A single stepped tunnel is bone-preserving in that it eliminates the need for two or more bone tunnels to create a bone bridge for tying knots for final fixation of soft tissue to bone.

The disclosure describes surgical adjustable loop constructs, suture loop mechanisms, all-suture knotless surgical repairs, and methods for securing a first tissue to a second tissue (for example, soft tissue to bone) with a tensionable construct including adjustable, knotless, flexible, closed loops.

An embodiment includes an all-suture tissue fixation construct. An implant construct can attach or re-attach normal anatomical structures, tissue to tissue, bone to bone, and/or bone to soft tissue. The construct can include suture implants attached in a knotless manner to at least one securing device for single tunnel tissue fixation.

A securing device can be a fixation device. A securing device can be a reinforcement device. A securing device can be in the form of any piece of material (such as a suture sheath, for example) that can be attached in a knotless manner to an all-suture implant for reinforced, knotless, tensionable, all-suture fixation. An all-suture implant can include two or more soft suture anchors that are connected/interconnected by at least one length of flexible coupler forming at least one knotless, continuous, flexible, closed adjustable loop and a flexible terminal end. A securing device can attach to the length of flexible coupler to provide secure fixation and reinforcement of the final repair. The construct can be shrunk by pulling on the terminal end of the flexible coupler so that the securing device is pulled into, and secured within, a stepped tunnel and the perimeter of the at least one knotless, continuous, flexible, closed adjustable loop decreases.

The flexible coupler enters and exits the soft suture anchors of the all-suture implant with ends that are spliced to themselves to form a plurality of knotless, continuous, flexible, closed adjustable loops having an adjustable perimeter. The construct can be shrunk when the terminal end is pulled. The construct allows the user (for example, surgeon) to control the tension of the flexible coupler on a first tissue (for example, soft tissue, meniscus, ligament, graft, or cartilage) to be attached to a second tissue (for example, bone) while the securing device allows secure fixation of the flexible coupler within the stepped bone tunnel.

A tissue repair system includes first and second fixation devices each in the form of a soft suture anchor and interconnected to each other by at least a length of a flexible coupler, the flexible coupler forming at least one knotless, continuous, flexible, closed adjustable loop with an adjustable perimeter and one terminal end; and a securing device attached to the flexible coupler connecting the first and second fixation devices. The system can further include a delivery device for deployment of the first and second fixation devices. A flexible coupler can be of either round and/or flat design. A flexible coupler can be suture and/or tape. A securing device can be a piece of material (of fixed or adjustable dimensions) that allows secure fixation of the flexible coupler within a bone tunnel and reinforcement of the repair. A securing device can be a suture material, a suture tape, a suture sheath, or any similar structure and/or material that allows the device to be introduced into a bone tunnel and secured against its walls. A tissue repair system can consist essentially of suture such as ultrahigh molecular weight polyethylene suture. A tissue repair system can provide bone-preserving, knotless, tensionable, all-suture tissue fixation.

A construct can be employed to attach or re-attach normal anatomical structures, i.e., a first tissue to a second tissue, such as soft tissue, tendon, ligament, meniscus, and/or bone, to each other and/or any combination of one another, by employing an all-suture, knotless, tensionable mechanism.

Methods of tissue repairs are also disclosed. A first tissue is approximated to a second tissue with a surgical construct that includes an all-suture, tensionable, knotless implant through a single stepped bone tunnel. The technique creates a bone-preserving, tensionable, knotless, all-suture repair.

### Description of Drawings

In the following, embodiments will be described by way of example, without limitation of the general inventive concept, on examples of embodiment with reference to the drawings.
FIG. 1 illustrates a surgical implant.
FIG. 2 illustrates a surgical assembly with the surgical implant of FIG. 1.
FIGS. 3-12 illustrate the surgical assembly of FIG. 2 employed in tissue repair.
FIGS. 13-20 illustrate the surgical assembly of FIG. 2 employed in another tissue repair.
FIGS. 21-24 illustrate additional views of the tissue repair of FIG. 20.

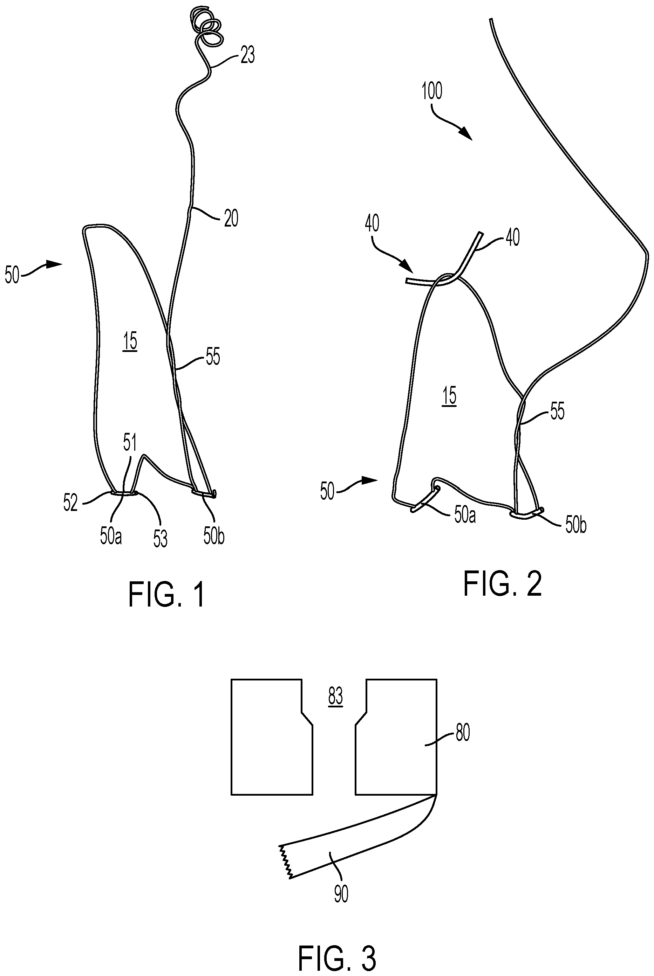

Referring now to the drawings, where like elements are designated by like reference numerals, FIGS. 1 and 2 illustrate structural elements of surgical assembly 100 (construct 100; implant 100; knotless construct 100; soft anchor meniscal repair 100; soft anchor single tunnel fixation system 100; system 100) formed of all-suture implant 50 (which includes implants or fixation devices 50a, 50b connected by flexible coupler 20) and at least one securing device 40. FIGS. 3-24 illustrate exemplary steps of tissue repair 200, 300 with surgical assembly 100.

As shown in FIGS. 1 and 2, assembly 100 includes all-suture implant 50 having two exemplary fixation devices 50a, 50b in the form of soft-suture anchors 50a, 50b that are knotlessly attached and interconnected to each other by at least one flexible coupler 20 and one or more loops. Each fixation device 50a, 50b can be in the form of a soft anchor (soft suture sheath, soft suture anchor, all-suture soft knotless anchor, implant) with a soft anchor sleeve 51 (sheath or tubular member 51) with two open ends 52, 53. At least one flexible coupler 20 extends through each of the soft anchor sleeve 51 (sheath 51) of each fixation device 50a, 50b.

Flexible coupler 20 can extend through the anchor sleeves in similar or different directions and/or orientations and/or locations. Flexible coupler 20 can extend through the whole length of each sleeve, i.e., enters one of the two open ends 52, 53 and exits other of the two open ends 52, 53, or only through a partial length of the sleeves. Details of an exemplary soft suture anchor with a soft anchor sleeve (sheath or tubular member) and flexible shuttling strands are set forth, for example, in US Patent 10,849,734 issued Dec. 1, 2020, entitled "Methods of Tissue Repairs," the disclosure of which is incorporated by reference in its entirety herein.

In an embodiment, fixation devices 50a, 50b can be formed essentially of suture. In an embodiment, fixation devices 50a, 50b can be formed of braided suture that can include fibers of ultrahigh molecular weight polyethylene (UHMWPE).

In an embodiment, fixation devices 50a, 50b can be formed of elastic suture. In an embodiment, fixation devices 50a, 50b can be formed of UHMWPE braided with polyester.

Flexible coupler 20 (tensionable construct 20; coupler 20; flexible material 20; flexible strand 20; flexible tape 20; suture 20) can be formed of one single continuous coupler in form of suture, either round and/or flat suture, for example, a suture strand or suture tape. Flexible coupler 20 has at least one terminal end (end 23) as shown in FIG. 1. Flexible coupler 20 can be a fixed length of suture and/or tape, such as suture tape.

In an embodiment, all-suture implant 50 of surgical assembly 100 can be formed by passing flexible coupler 20 through at least a portion of the body of one of the two fixation devices 50a, 50b (for example, first soft-anchor 50a, or proximal anchor 50a) and then through at least a portion of the body of the other of the two fixation devices 50a, 50b (for example, second soft-anchor 50b, or distal anchor 50b). One terminal end is then spliced through itself to form at least one splice 55 and at least one flexible, closed, knotless, adjustable loops 15 adjacent terminal end 23. The passes and splices can be formed with suture passers and/or shuttle/pull devices and/or suture passing instruments such as needles, Fiber-Link^{™} loops, nitinol loops, or any suture passing device that includes an eyelet/loop for passing the flexible coupler 20.

FIG. 2 illustrates assembly 100 which consists of all-suture implant 50 (two soft suture sheaths 50a, 50b connected by way of a single continuous suture 20 which loops through both to create a means for knotless tensioning) and at least one securing device 40 (fixation device 40; bridge 40; bone-tunnel filler 40; fastener 40; reinforcement 40; securing mechanism 40). Securing device 40 can be a soft-body fixation device in the form of a piece of material (of fixed or adjustable dimensions) that allows attachment to the flexible coupler 20 and secure fixation of the flexible coupler 20 within a bone tunnel. Securing device 40 can be suture, tape, sheath, or similar structures of various dimensions and/or configurations depending on the dimensions of the tunnel to be filled/inserted into. In an exemplary-only embodiment, securing device 40 can be a suture sheath 40 that allows the device to be passed through and looped around loop 15 of flexible coupler 20, and then introduced into a stepped bone tunnel and secured against its walls. Assembly 100 (tissue repair system 100) can consist essentially of suture such as ultrahigh molecular weight polyethylene suture. Assembly 100 (tissue repair system 100) can consist essentially of elastic suture.

All-suture implant 50 and assembly 100 can be shrunk when terminal end 23 of coupler 20 is pulled to decrease the perimeter of at least one of the flexible, closed, knotless, adjustable loop 15. If necessary, multiple securing devices 40 and/or additional strands and/or materials can be attached to implant 50 by passing them through loop 15. Additional strands can be FiberWire^{®} suture, TigerWire^{®} suture, FiberTape^{®} suture tape, among many others.

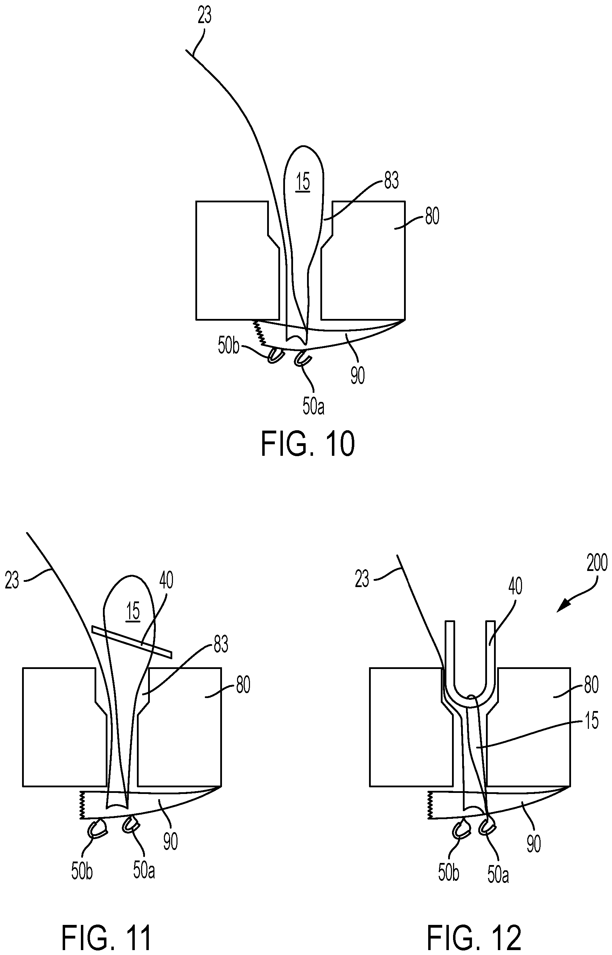

Reference is now made to FIGS. 3-12 which illustrate steps of an exemplary method of tissue repair 200 (FIG. 12) with system 100, for attaching a first tissue to a second tissue, for example, soft tissue 90 to bone 80.

FIG. 3: Create stepped tunnel 83 within bone 80 to repair soft tissue 90. Using a drill guide, drill a pilot hole until desired depth is reached depending on thickness of bone 80 (bone fragment 80). A stepped or tapered drill can be used to form stepped hole 83 in bone 80.

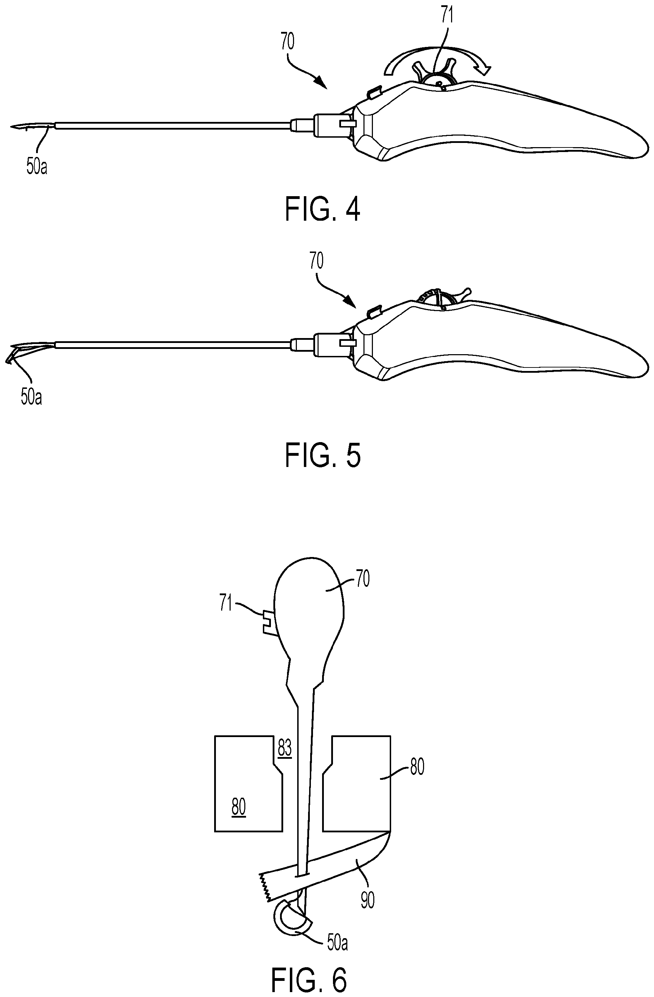

FIGS. 4 and 5: An anchor inserter 70 (anchor delivery device 70; delivery needle 70; inserter 70) is employed to insert one of the two suture-based implants 50a, 50b, for example, implant 50a, into soft tissue 90. Rotate wheel 71 clockwise to deploy first implant 50a.

FIG. 6: Insert anchor delivery device 70 through tunnel 83 and pierce soft tissue 90 as shown. Deploy implant 50a on the far side of soft tissue 90. FIG. 6 illustrates first implant 50a deployed and with instrument 70 piercing soft tissue

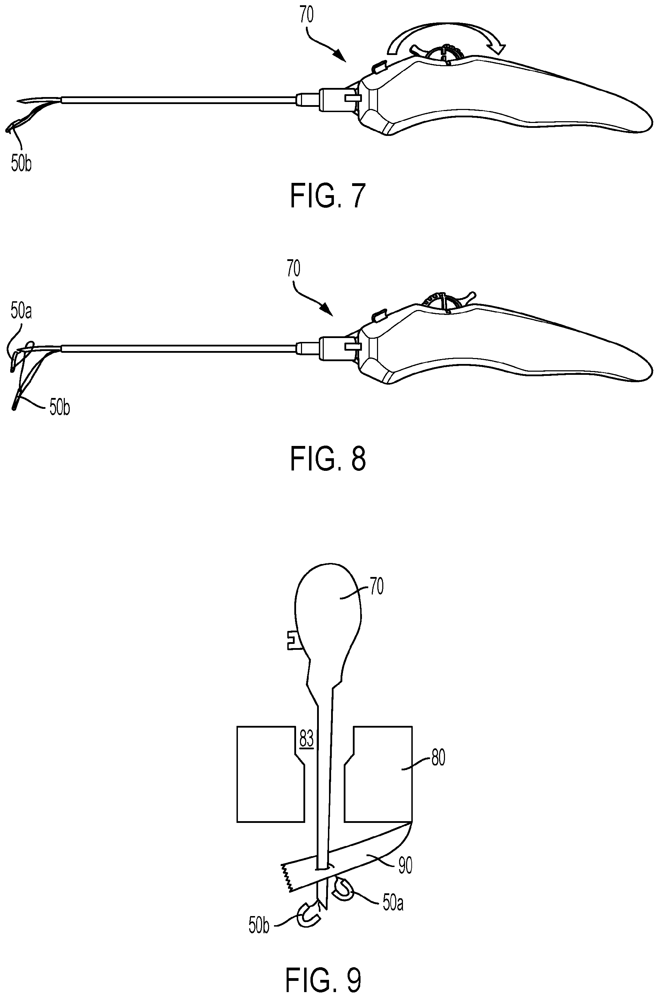

FIGS. 7 and 8:For deployment of second implant 50b, remove needle tip of inserter 70 from soft tissue 90 and pierce through soft tissue 90 again, at a second location, approximately 1 to 3mm away from first implant 50a.

FIG. 9: Remove anchor delivery device 70 from soft tissue 90 and pierce the soft tissue 90 approximately 1 to 3mm away from first implant. Deploy second implant 50b on far side of soft tissue at the second location. FIG. 9 illustrates anchor delivery device 70 passed through stepped tunnel 83 and with both implants 50a, 50b deployed.

FIG. 10: Remove anchor delivery device 70 from the tunnel 83 leaving the two implants 50a, 50b on the far side of the tissue 90 while the loop 15 and tensioning tails 23 extend through the bone tunnel. Remove anchor delivery device 70 leaving implants 50a, 50b deployed and knotless loop 15 through stepped tunnel 83.

FIGS. 11 and 12: Slide suture sheath 40 through loop 15 and pull tensioning tail 23 to reduce knotless loop 15. This action pulls the sheath 40 into bone tunnel 83 and creates a suture bridge for compressing the soft tissue 90 back to bone 80. Sheath 40 acts as a "plug" for bone tunnel 83 as well as a "bridge" for tissue fixation. If desired, ends of sheath 40 as well as terminal end 23 can be cut flush with bone 80.

Current techniques for attaching soft tissue to bone require drilling two tunnels for creating a boney bridge to tie knots over for final fixation of soft tissue to bone. As detailed above, the present disclosure eliminates the need to create two (or more) bone tunnels. To facilitate a single stepped tunnel technique, the bone bridge is replaced with a suture sheath 40 which creates its own bridge for both tensioning and final fixation. Additionally, the final construct 200 is knotless and all-suture, eliminating the need for hard anchors (i.e., plastic or metal fixation devices) and the need to tie knots for final fixation.

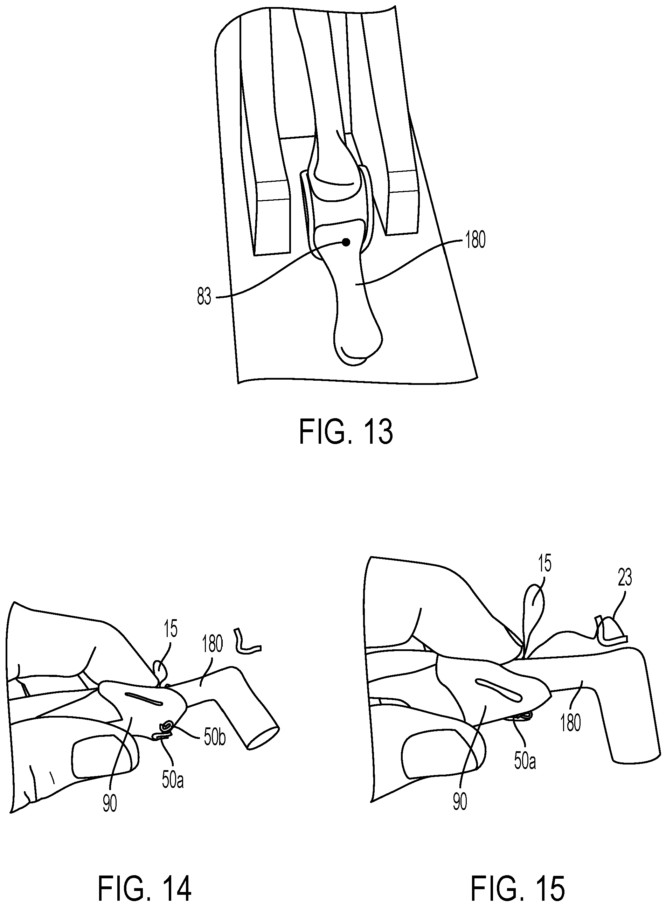

FIGS. 13-24 illustrate an exemplary plantar plate repair with assembly 100. Current plantar plate repair techniques and products on the market require either two tunnels to be drilled or one large bi-cortical tunnel. Final fixation with the current techniques and products is completed by either tying knots or using hard-body fixation devices such as interference screws or suture anchors. As explained above and as also detailed below, the devices, systems, and techniques of the present disclosure create a bone-preserving, all-suture, knotless, and tensionable technique.

FIG. 13: A single stepped tunnel 83 is formed in proximal phalanx 180. A stepped or tapered drill can be used to form stepped hole 83 in phalanx 180. If desired and necessary after osteotomy, a K-wire (such as a vertical 1.6 mm K-wire) can be employed to temporarily fix the metatarsal.

FIGS. 14 and 15: Insert anchor delivery device 70 (delivery needle 70; inserter 70) through tunnel 83 and pierce soft tissue 90. Deploy first implant 50a on the far side of soft tissue 90. For deployment of second implant 50b, remove needle tip of inserter 70 from soft tissue 90 and pierce through soft tissue 90 again, at a second location, approximately 1 to 3mm away from first location of the first implant 50a. Anchor delivery device 70 is removed from tunnel 83 leaving the two implants 50a, 50b on the far side of the tissue 90 while the loop 15 and tensioning tails 23 extending through the stepped tunnel 83.

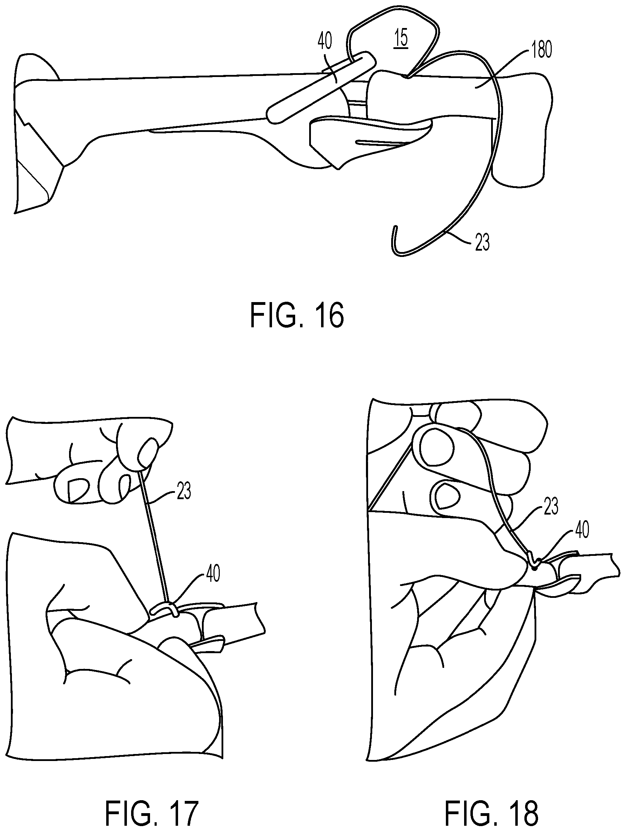

FIG. 16: Securing device 40 (suture sheath 40) is passed and looped through knotless, adjustable, continuous, tensionable loop 15.

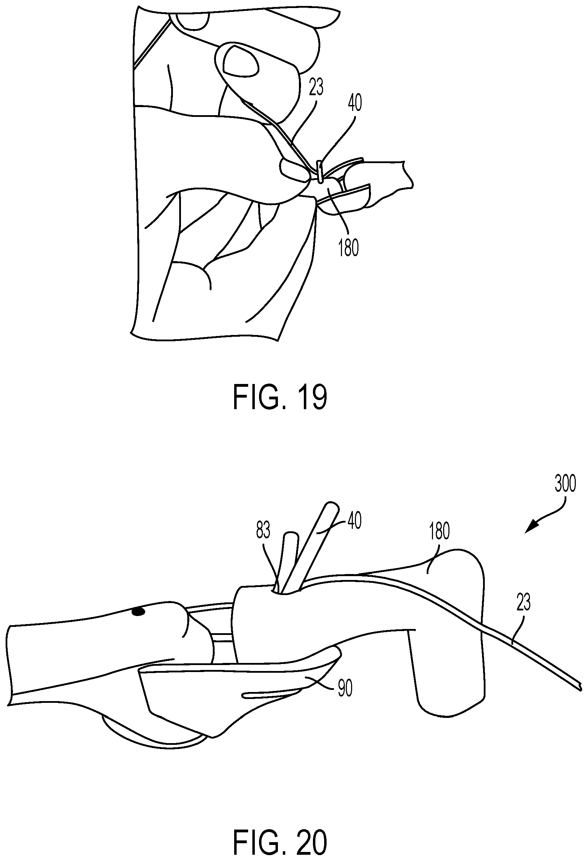

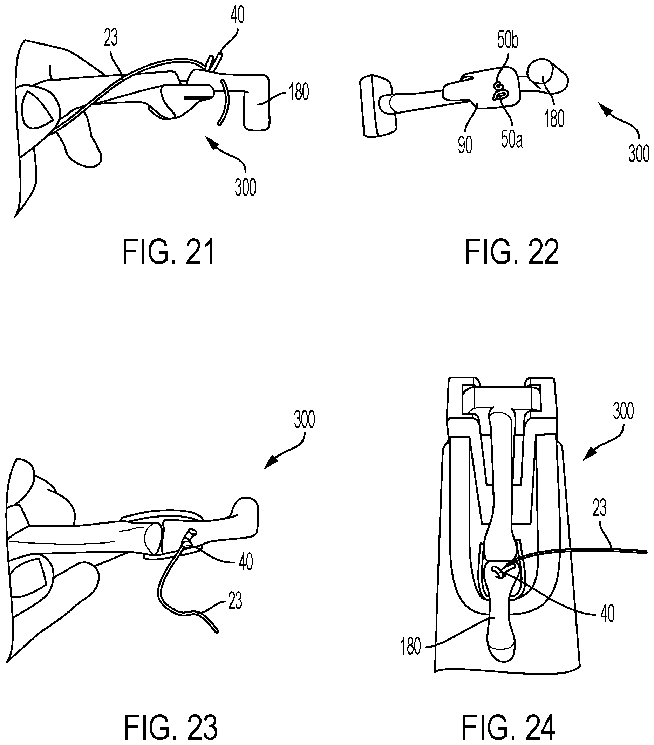

FIGS. 17-20: Once suture sheath 40 is passed through loop 15, pull on tensioning tail 23 to reduce the perimeter of knotless loop 15. This action pulls the sheath 40 into stepped bone tunnel 83 and creates a suture bridge for compressing the soft tissue 90 back to bone 180. Sheath 40 acts as a "plug" for bone tunnel 83 as well as a "bridge" and reinforcement for plantar plate repair 300 (FIG. 20). If a K-wire is employed, the K-wire can be removed before final fixation (additional fixation devices such as screws can be employed for osteotomy repair). FIGS. 21-24 illustrate additional views of the plantar plate repair 300 of the present disclosure.

In embodiments described herein, knotless, all-suture soft tissue fixation can provide soft suture anchor fixation in a simple manner, with fewer steps, bone preservation, and overall increased and reinforced fixation.

Implant 50 and assembly 100 can be employed with one or more biologics, for example, soft tissue-to-bone biologics. Various medicinal and/or therapeutic agents, for example, antiseptics, antibiotics, drugs, pharmaceutical agents, hormones, and growth materials (for example, autogenous growth factors such as platelet-rich plasma (PRP), or autologous factors) among many others can be added at the repair site to aid in the wound closure and overall healing.

The benefits of all-suture implant 50 and assembly 100 to the patient include no prominent or bulging metal hardware left in the body that can cause soft tissue irritation; less scar tissue formation; immediate enclosure of the single bone tunnel allowing faster healing; increased bone preservation; and reinforced tissue repair. All-suture implant 50 and assembly 100 eliminate the large "knots" palpable under the skin of the prior art designs, allowing for specific tailoring of height relative to the bone surface while preserving the bone mass.

All-suture implant 50 (suture-based implant 50) and surgical assembly 100 of FIGS. 1-24 are self-locking, adjustable, knotless, tensionable loop constructs. Assembly 100 can be a suture/soft anchors construct in the form of an orthopedic implant construct which may be utilized to attach or re-attach a first tissue to a second tissue, for example, normal anatomical structures, bone to bone, tissue to tissue, and/or bone to tissue, among others. The construct includes a single strand of suture run through two soft suture anchors to create a tensionable, self-locking, adjustable loop, and a securing device (suture sheath) passed through the loop to anchor and reinforce the construct within a single, stepped bone tunnel. The construct can be provided assembled, disassembled, as part of a kit, with or without additional instruments (such as suture passers or delivery devices), fixation devices and/or flexible couplers, to facilitate passing through tunnels and/or bone sockets and/or openings.

All-suture-based, arthroscopic tissue repair devices/constructs of the present disclosure can be employed for any tissue positioning and/or tissue adjustment applications, for example, in fixation of bone to bone (such as small joint applications, or acromioclavicular joint fixation techniques) which employ two fixation devices (for example, two all-suture anchors) joined by a continuous suture loop formed by a continuous flexible coupler. In exemplary embodiments only, assembly 100 of the present disclosure can be employed in a method of bunion repair and/or in a method of Lisfranc repair. Similarly, assembly 100 can be employed in any method of fixation of bone.

The all-suture implant 100 with the securing mechanism 40 can be utilized in surgical procedures such as rotator cuff repair, Achilles tendon repair, patellar tendon repair, ACL/PCL reconstruction, hip and shoulder reconstruction procedures, AC joint reconstruction, syndesmosis reconstruction, quad/patellar tendon rupture repair, hallux-valgus repair, proximal and/or distal biceps tendon repair, humerus and radius repair, and any other tendon repair to bone, among many others, all conducted in a knotless manner.

A surgical assembly 100 includes a plurality of fixation devices 50a, 50b; a knotless, adjustable, self-locking tensionable construct 20 loaded on the fixation devices 50a, 50b; and a securing device 40. The tensionable construct 20 includes at least one flexible coupler 20 having a terminal end 23; at least one closed, adjustable, continuous, flexible loop 15; and at least one splice 55. Fixation devices 50a, 50b are all-suture soft anchors. Securing device 40 can be a suture sheath formed essentially of suture. Surgical assembly 100 can connect first tissue 80 to second tissue 90. First tissue 90 can be a soft tissue and second tissue 80, 180 can be bone. First and second fixation devices 50a, 50b can reside and be secured on the far side of soft tissue 90. Securing device 40 is passed and looped through loop 15. Terminal end 23 is pulled to decrease the length and perimeter of flexible, adjustable, closed, knotless loop 15, and to secure the securing device 40 within stepped tunnel 83 formed within second tissue 80, 180. Surgical assembly 100 can consist essentially of suture. Surgical assembly 100 can consist essentially of elastic suture.

A method of tissue repair 200, 300 comprises inter alia: passing a flexible coupler 20 through sheaths of two fixation devices 50a, 50b, to form a knotless, tensionable, self-locking, adjustable, all-suture implant 50 with at least one loop 15 and at least one splice 55; forming a stepped tunnel 83 within a first tissue 80, 180; securing the first and second fixation devices 50a, 50b to a second tissue 90 so that the at least one loop 15 extends within and out of the stepped tunnel 83; passing a securing device 40 through the at least one loop 15 to form assembly 100; and securing the securing device 40 in the first tissue 80, 180. The method can further include tensioning the construct by pulling on terminal end 23 of flexible coupler 20; and securing the securing device within a stepped tunnel formed within the first tissue 80, 180. The securing device 40 can be a suture sheath. Assembly 100 can consist essentially of suture. The tissue repair can be a soft tissue-to-bone repair. The tissue repair can be a plantar plate repair.

A method of knotless repair 200, 300 comprises: forming a single stepped tunnel 83 through a first tissue 80, 180; securing first and second fixation devices 50a, 50b of all-suture implant 50 to a second tissue 90 to be attached to the first tissue 80, 180; passing a securing device 40 through a loop 15 formed by a single, continuous, flexible coupler 20 attaching the first and second fixation devices 50a, 50b; and pulling on a terminal end 23 of flexible coupler 20 to tension repair 200, 300, approximate the second tissue 90 to first tissue 80, 180, and fixate the securing device 40 within stepped tunnel 83. The first tissue 80 can be bone, and the second tissue 90 can be soft tissue.

Flexible coupler 20 and securing device 40 can be formed of various flexible materials and strands such as round suture, flat suture, ribbon, or flat tape (for example, suture tape) or combination of suture and tape. Exemplary materials can include suture, silk, cotton, nylon, polypropylene, polyethylene, ultrahigh molecular weight polyethylene (UHMWPE), polyethylene terephthalate (PET), and polyesters and copolymers thereof, or combinations thereof. Flexible strand/coupler 20 and securing device 40 can have cross-sections of various forms and geometries, including round, oval, rectangular, or flat, among others, or combination of such forms and geometries. In an exemplary embodiment only, both flexible coupler 20 and securing device 40 can be formed of a high strength suture material such as FiberWire^{®} suture, sold by Arthrex, Inc. of Naples, Fla., and described in US 6,716,234, the disclosure of which is incorporated by reference herein. FiberWire^{®} suture is formed of an advanced, high-strength fiber material, namely ultrahigh molecular weight polyethylene (UHMWPE), sold under the tradenames Spectra^{®} (Honeywell International Inc., Colonial Heights, Va.) and Dyneema^{®} (DSM N.V., Heerlen, the Netherlands), braided with at least one other fiber, natural or synthetic, to form lengths of suture material. Flexible coupler 20 and securing device 40 can be braided or multi-filament suture such as FiberTape^{®} suture tape (as disclosed in US 7,892,256, the disclosure of which is incorporated in its entirety herewith). If suture tape is employed, the tape can have sections with different tapers (for example, 2 or 3 sections of gradual tapers or gradual widths) to facilitate easy formation of the splice regions 55 and loops 15. For example, splice region 55 can be round suture while loops 15 can be formed of flat sections.

Flexible coupler 20 and securing device 40 can be also formed of a stiff material, or combination of stiff and flexible materials, particularly for the regions of the couplers that are passed/spliced through the body of the coupler and depending on whether they are employed with additional fixation devices. Various regions, or sections of flexible coupler 20 and securing device 40 can be coated and/or provided in different colors for easy manipulation during the surgical procedure. Flexible coupler 20 and securing device 40 can be provided with tinted tracing strands, or otherwise contrast visually with the sheath of the soft suture anchors 50a, 50b, which remains a plain, solid color, or displays a different tracing pattern, for example. Easy identification of suture in situ is advantageous in surgical procedures, particularly during arthroscopic surgeries, endoscopic and laparoscopic procedures.

Various elements of assembly 100 can be also coated (partially or totally) with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the suture or tape, knot security, pliability, handleability or abrasion resistance, for example. If desired, at least a region of the flexible strands/coupler 20 and securing device 40 can be coated, impregnated, or otherwise stiffened with a material such as plastic, for example.

The term "high strength suture" is defined as any elongated flexible member, the choice of material and size being dependent upon the particular application. For the purposes of illustration and without limitation, the term "suture" as used herein may be a cable, filament, thread, wire, fabric, or any other flexible member suitable for tissue fixation in the body.

A surgical repair consisting essentially of two soft suture anchors secured on a surface of a first tissue to be attached to a second tissue, and a suture sheath knotlessly attached to the two soft suture anchors and secured within a stepped tunnel formed within the second tissue.

A method of tissue repair, comprising:
- inserting through a first tissue and through a second tissue a first soft anchor, wherein the first soft anchor is knotlessly connected to a second soft anchor by at least one flexible coupler forming at least one knotless, adjustable, continuous, flexible loop;
- securing the first soft anchor in the second tissue;
- securing the second soft anchor in the second tissue;
- passing a securing device through the at least one knotless, adjustable, continuous, flexible loop; and
- securing the securing device in the first tissue.

The above method further comprising forming a stepped tunnel into the first tissue, pulling the securing device into the stepped tunnel, and securing the securing device into the stepped tunnel.

The above method may further comprise:
- passing at least once the at least one flexible coupler through a first tubular sheath of the first soft anchor;
- passing at least once the at least one flexible coupler through a second tubular sheath of the second soft anchor to form the at least one flexible, closed, continuous loop connecting the first soft anchor to the second soft anchor;
- securing the first soft anchor at a first location in the second tissue and securing the second soft anchor at a second location in the second tissue, wherein the first location is different from the second location; and
- pulling on a terminal end (23) of the flexible coupler to pull the securing device into the first tissue.

The above method, wherein the securing device may be pulled and fixated within a stepped tunnel formed within the first tissue.

The above method, wherein the first tissue may be bone and the second tissue may be soft tissue, ligament, or graft.

The above method, wherein each of the first and second soft anchors may be an all-suture anchor formed of a single suture material, wherein the flexible coupler may be elastic suture, and wherein the securing device may be a suture sheath.

## Claims

1. A knotless, all-suture, surgical construct (100) comprising:
a first soft anchor knotlessly connected to a second soft anchor by a flexible coupler; and
a securing device (40) attached to the flexible coupler (20).

2. The surgical construct (100) of claim 1, wherein the flexible coupler (20) passes at least once through a first tubular sheath of the first soft anchor and at least once through a second tubular sheath of the second soft anchor to form at least one closed, continuous, flexible loop (15) with an adjustable perimeter.

3. The surgical construct (100) of any of the previous claims, wherein the securing device (40) is passed through and looped around the at least one closed, continuous, flexible loop (15) with an adjustable perimeter.

4. The surgical construct (100) of any of the previous claims, wherein the flexible coupler (20) enters the first tubular sheath at a first location, extends within the first tubular sheath and exits the first tubular sheath at a second location, wherein the second location is different from the first location.

5. The surgical construct (100) of any of the previous claims, wherein the flexible coupler (20) enters the second tubular sheath at a first location, extends within the second tubular sheath and exits the second tubular sheath at a second location, wherein the second location is different from the first location.

6. The surgical construct (100) of any of the previous claims, wherein the flexible coupler (20) is spliced through itself at one or more locations.

7. The surgical construct (100) of any of the previous claims, wherein each of the first soft anchor and the second soft anchor is an all-suture anchor, the flexible coupler (20) is round suture, and the securing device (40) is a soft-body fixation device, and wherein the securing device (40) may be a suture, suture tape, suture tube, or suture sheath.

8. The surgical construct (100) of any of the previous claims, wherein the construct consists essentially of suture and/or
wherein the flexible coupler (20) consists essentially of elastic material.

9. The surgical construct (100) of any of the previous claims, wherein the first soft anchor and the second soft anchor are secured within or on a first tissue, and wherein the securing device (40) is secured within a second tissue.

10. The surgical construct (100) of any of the previous claims, wherein the securing device (40) is secured within a stepped tunnel formed within the second tissue.

11. A surgical assembly for tissue repairs, comprising:
a first fixation device and a second fixation device, each consisting essentially of suture;
a flexible coupler (20) passed through the first fixation device and through the second fixation device, the flexible coupler (20) forming in a knotless manner at least one splice (55) and at least one continuous, adjustable,
tensionable loop (15) extending between the first fixation device and the second fixation device; and
a securing device (40) passed through and looped around the at least one continuous, adjustable, tensionable loop (15).

12. The surgical assembly of claim 11, further comprising a delivery needle for sequentially deploying the first fixation device and the second fixation device within a first tissue to be attached to a second tissue, and/or wherein the securing device (40) is secured within a stepped tunnel formed within the second tissue.

13. The surgical assembly of any of claims 11 to 12, wherein, when the flexible coupler (20) is pulled,
either a perimeter and a length of the at least one continuous, adjustable, tensionable loop (15) decreases and tensions the surgical assembly,
or the securing device (40) is pulled into the stepped tunnel and fixated therein.

14. The surgical assembly of any of claims 11 to 13, wherein each of the first fixation device and the second fixation device includes a tubular sheath with two open ends.

15. The surgical assembly of any of claims 11 to 14, wherein the tissue repair is rotator cuff repair, AC joint repair, syndesmosis repair, Achilles tendon repair, patellar tendon repair, ACL/PCL reconstruction, hip and shoulder reconstruction, AC joint reconstruction, syndesmosis reconstruction, quad/patellar tendon rupture repair, plantar repair, or hallux-valgus repair or wherein the tissue repair is plantar plate repair.
